Europäisches Patentamt

European Patent Office

Office européen des brevets

(11) Publication number: **0 238 294**
**A2**

# (12) EUROPEAN PATENT APPLICATION

(21) Application number: **87302244.6**

(22) Date of filing: **17.03.87**

(51) Int. Cl.⁴: **A 61 M 5/16**

(30) Priority: **18.03.86 GB 8606626**

(43) Date of publication of application:
**23.09.87 Bulletin 87/39**

(84) Designated Contracting States:
**BE DE FR GB NL SE**

(71) Applicant: **Travenol Laboratories Limited**
**Caxton Way**
**Thetford Norfolk (GB)**

(72) Inventor: **Hellen, Graham**
**29 Brown Street West**
**Colne Lancashire BB8 9ND (GB)**

**Howarth, Michael John**
**19 Mild May Terrace**
**Hartley Wintney Hampshire (GB)**

**Hayes, Harry**
**Linden Dene Marie Drive**
**Thelwell Warrington Cheshire WP4 3JS (GB)**

(74) Representative: **MacGregor, Gordon**
**ERIC POTTER & CLARKSON 14 Oxford Street**
**Nottingham, NG1 5BP (GB)**

(54) **Parenteral administration apparatus.**

㊼ The apparatus comprises a filter chamber (l3) having an upline end (ll) for connection with a blood or parenteral solution container, and a downline end (l5) connected to a drip chamber (l4) which, in turn, is connected in use to a patient.

The filter chamber houses first (l20) and second (l25) filters of the sleeve or pouch type, the filters being secured about the upline end, so that liquid passes into the first filter for collection of macroaggregates, then into the second filter for collection of microaggregates and finally into the body of the chamber to reach the drip chamber.

Fig. 2

# Description

## PARENTERAL ADMINISTRATION APPARATUS

This invention relates to parenteral administration apparatus for parenteral infusion of a liquid to a patient. The liquid may be a parenteral solution, blood, or blood plasma.

A parenteral administration set comprises a conduit having a hollow spike at one end for connection with a solution container, means at the opposite end for connection with a patient and chamber means between the spike and the needle, the chamber means including a filter chamber and a drip chamber. The means for connection with a patient usually comprises a connector for connection to a length of tubing with a hollow needle at one end.

Most countries have regulations governing the minimum size of particles which should be filtered, the particle size being greater for blood than for parenteral solution. In the United Kingdom it is usual to provide a 170 micron filter for filtering blood and a 15 micron filter for filtering parenteral solution. It is a common practice, however, for infusing blood, to add a further 40 micron filter to the administration set, connected between a blood container and the administration set. Microaggregates form in old stored blood and it is important to filter out these particles. A 170 micron filter is suitable for fresh blood and filters out macroaggregates, but microaggregates are allowed to pass. The additional 40 micron filter removes both macroaggregates and microaggregates, but renders the 170 micron filter, in the filter chamber, redundant.

An administration set is a closed and sterile unit and communication with a blood container is carried out in a sterile manner. The hollow spike is sealingly engaged in a tubular port of the container as it is pushed into a position in which a membrane is pierced by the spike to effect communication with the blood container. The use of an additional filter, however, prevents this closed, sterile connection being effected. The hollow spike has to be connected to the filter, after which the filter is connected to the blood container. The added filter also makes priming of the set more difficult after connection to the blood container.

It would be advantageous to use a 40 micron filter in the filter chamber instead of the 170 micron filter. This would avoid the "open" connection to the blood container. This is not possible, however, using a filter chamber of conventional size, because the filter would be too small and liable to clogging by the combined macroaggregates and microaggregates. "Dumping", i.e. the passage of particles through the filter due to their accumulation on the filter may then occur.

To make an administration set with a specially large filter chamber and large 40 micron filter would very substantially add to the manufacturing costs and expense of the administration set. In one commercially available set, for example, the filter chamber and the drip chamber are formed from a flexible plastics tube which is heat sealed to conduits to define the chambers. This allows the set to be made cheaply, but the filter chamber has a small diameter relative to that of the further filter commonly added to such a set. Only a small filter can, therefore, be accommodated in the chamber.

The present invention provides parenteral administration apparatus, which can be cheaply made by present commercial methods, but which permits the filtering of microaggregates from blood without use of an additional filter. It is envisaged that the administration set of the present invention may by suitable for filtering both blood and parenteral solution, so that special sets would not have to be made for the different uses.

In accordance with this invention, parenteral administration apparatus comprises a conduit having an upline end for connection with a liquid container, a downline end for connection to a patient, and chamber means between said ends and including a filter chamber and a drip chamber downline of the filter chamber, the filter chamber housing a first filter for filtering macroaggregates and passing microaggregates and the chamber means housing a second filter downstream of the first filter for filtering microaggregates.

The pore size of the first filter may be from 120 to 350 microns, usually 120 to 240 microns and, preferably, 140 to 200 microns.

The pore size of the second filter is preferably less than 110 microns, and may be 10 to 70 microns, or, advantageously, 15 to 50 microns.

The total effective filter area in the chamber means is advantageously at least 10 cm² and preferably at least 30 cm².

A third filter may be included in the chamber means, e.g. to filter out the larger macroaggregate particles with the first filter serving to filter out the smaller macroaggregate particles.

The filters may be made of a blood compatible polyamide or polyester material and the chamber means may be made from polyvinyl chloride.

The second filter may be provided in either the filter chamber, or the drip chamber and may surround the first filter in the filter chamber.

In the preferred embodiment, the first filter is a pouch secured with its mouth in communication with the upline end of the filter chamber so as to collect macroaggregates in the pouch and the second filter is also a pouch, secured about the first filter so as to enclose the latter, so that microaggregates collect in the second filter. This arrangement provides a synergistic effect, so that it is possible to use a pore size for the second filter larger than the particles held in the combined filter.

In one described embodiment, the second filter has a pore size of about 80 microns, yet is as effective in retaining microaggregates as a 40 micron filter used alone.

The pore size of the second filter may be 50 to 110 microns and, preferably, is 70 to 95 microns.

Reference is now made to the accompanying

drawings, wherein:-

Figure 1 is a sectional view of a chamber portion of a parenteral administration set according to the invention; and

Figure 2 is a similar view of another embodiment of the invention.

The administration set includes, in addition to the chamber portion illustrated, a hollow spike for connection to a liquid container at an upline end of the set and a connector at a downline end of the set. The liquid container is usually a collapsible bag having a port which sealingly receives the hollow spike. The connector is connected to a length of tubing having a hollow needle at one end for connection with a patient. Non-collapsible containers will necessitate use of a separate air vent.

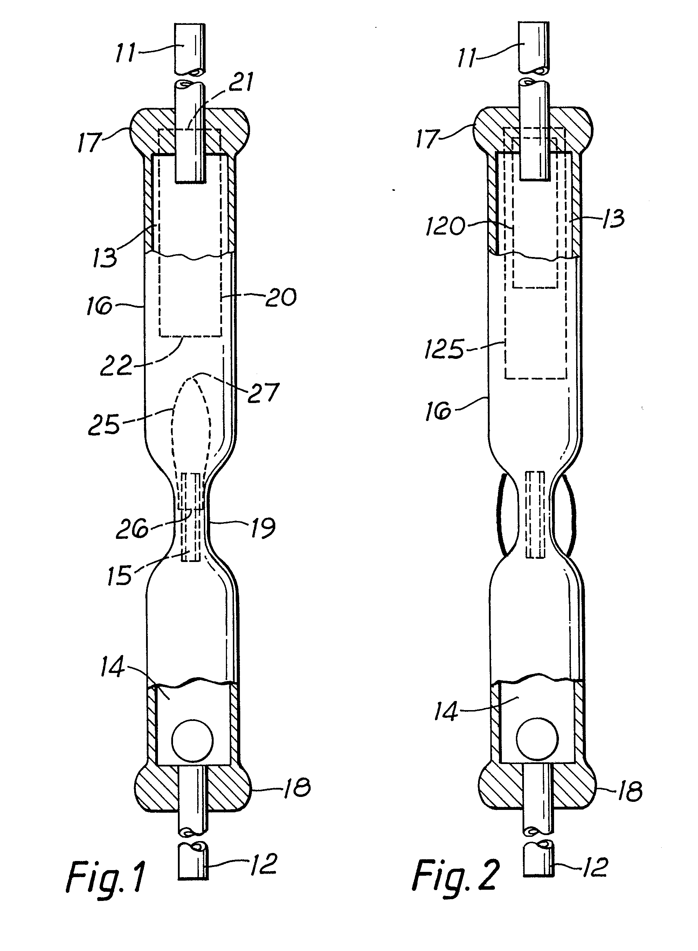

With reference to Figure 1, the hollow spike is connected to an upline conduit 11 and the connector is connected to a downline conduit 12. The conduits are sealingly connected respectively to a filter chamber 13 and a drip chamber 14 and the two chambers are sealingly connected by a tube 15. The whole set is closed and is internally sterile, the spike and connector being sealingly closed by removable means.

In the specific embodiment illustrated, the two chambers 13, 14 are constructed from a single length of flexible tubing 16 composed of thermoplastic material, such as a polyvinyl chloride formulation. Each end part of the tubing 16 receives an end part of a corresponding one of the conduits 11, 12 and is heat-sealed around the conduit by heat seal 17, 18 respectively. The flexible tubing 16 is also heat-sealed at 19, generally mid-way between its ends, about the tube 15, which is of small diameter relative to the conduits. The two chambers 13, 14 are thereby defined.

The filter chamber 13 houses a first filter 20 of a mesh size to pass microaggregates, but filter out macroaggregates (170 microns in this example). The filter illustrated is of pouch form with its mouth 21 sealed between the upline conduit 11 and the flexible tubing 16 by the heat seal 17. The opposite end of the filter to the mouth is closed at 22.

The filter chamber 13 also houses a relatively small second filter 25 of a mesh size to filter out microaggregates (40 microns in this example). This filter 25 is also of pouch form, in this example, and has its mouth 26 sealed between the downline conduit 12 and the flexible tubing 16 by the heat seal 19. The second filter extends towards the first filter and is closed at its end 17 opposite to its mouth 26. This second filter may include support means to prevent collapse of the filter, since, in use, liquid passes from the exterior to the interior of the pouch. Filters of the pouch type arranged for operation in this manner are often referred to as "candle" filters.

In use, blood, or other liquid passes into the first filter 20 from the upline conduit 11 and then passes out of the first filter and through the second filter to the tube 15. The blood drips from the tube into the drip chamber, collects in the drip chamber and passes through the downline conduit 12 to be infused in a patient.

The macroaggregates are filtered out of the blood by the first filter and are retained in the first filter and the microaggregates are filtered out of the blood by the second filter and collect on the second filter and at the bottom of the filter chamber around the second filter.

The construction described is of commercially available construction, except for the addition of the microaggregate filter. The administration set can, therefore, be made in conventional manner without increasing the size of the filter chamber.

It has been found that by using two separate filters in this manner, clogging of the microaggregate filter is avoided and effective filtering of macroaggregates and microaggregates from old stored blood is achieved.

In a modification, the second filter 25 is provided in the drip chamber 14 instead of the filter chamber 13. The second filter is sealed around the downline conduit 12 by the heat seal 18 and is in the form of a "candle" filter. Filters of other types may, of course, be used.

Referring to Figure 2, there is shown another embodiment of the invention similar to that described with respect to Figure 1. The construction is identical to that of Figure 1 except as regards the first and second filters 120 and 125 respectively.

The first, macroaggregate filter 120 is again of the pouch type and is sealed around the upline conduit 11 by the heat seal 17. The filter is, however, shorter than that shown in Figure 1.

The second, microaggregate filter 125 is also of pouch form, but is longer than the first filter 120. This second filter surrounds the first filter and is also sealed at its mouth about the upline conduit 11 by the heat seal 17.

In this embodiment, blood passes from the first filter directly into the second filter, macroaggregates collect in the first filter and microaggregates collect in the second filter.

This arrangement provides a synergistic effect and the filtering ability of this combination filter is greater than that of two such filters used separately. In the described embodiment, the first, inner filter has a pore size of 190 microns and the second, outer filter has a pore size of 80 microns. Despite the relatively large size of the pores of the second filter, the combination filter retains microaggregates down to 40 microns.

## Claims

1. Parenteral administration apparatus including a filter chamber having an upline end for connection with a liquid container, a downline end for connection to a patient, a first filter in the chamber for filtering macroaggregates and passing microaggregates and having a pore size of 120 to 350 microns and a second filter downstream of the first filter for filtering microaggregates.

2. A modification of the parenteral administration apparatus of Claim 1, including a drip chamber downline of the filter chamber, wherein the second filter is housed in the drip chamber instead of the filter chamber.

3. Parenteral administration apparatus according to Claim I or 2, wherein the first filter comprises a pouch whose mouth is in communication with said upline end for receiving liquid within the pouch and retaining macroaggregates within the pouch.

4. Parenteral administration apparatus according to Claim I or 3, wherein the second filter comprises a pouch whose mouth is in communication with an outlet opening from the filter chamber, the outlet opening communicating with the drip chamber, whereby microaggregates are retained in the filter chamber around the second filter.

5. Parenteral administration apparatus according to Claim 3, wherein the second filter comprises a pouch whose mouth is sealed around the first filter with the first filter enclosed within the second filter, whereby microaggregates are retained in the second filter.

6. Parenteral administration apparatus according to Claim 5, wherein the pore size of the second filter is 50 to II0 microns.

7. Parenteral administration apparatus according to Claim 6, wherein the pore size of the second filter is 70 to 95 microns.

8. Parenteral administration apparatus according to any one of Claims I to 4 wherein the pore size of the second filter is II0 microns or less.

9. Parenteral administration apparatus according to Claim I, wherein the pore size of the first filter is I20 to 240 microns.

I0. Parenteral administration apparatus according to Claim 2, wherein the pore size of the first filter is I40 to 200 microns.

**Fig.1**

**Fig.2**